# FASCICULE DE BREVET EUROPEEN

(11) **EP 0 680 299 B1**
(45) Date de publication et mention de la délivrance du brevet: **29.04.1998**
(21) Numéro de dépôt: 93904071.3
(22) Date de dépôt: 11.01.1993
(51) Int. Cl.: A61F 13/02

(54) **PROCEDE DE FABRICATION D'UN PANSEMENT A BORDS AMINCIS**
VERFAHREN ZUR HERSTELLUNG EINES MIT VERDÜNNTEN RÄNDERN VERSEHENEN VERBANDES
METHOD FOR PRODUCING A THIN-EDGED PLASTER

(30) Priorité: 29.01.1992 FR 9200936
(43) Date de publication de la demande: 08.11.1995
(73) Titulaire: LABORATOIRES D'HYGIENE ET DE DIETETIQUE L.H.D. Société Anonyme dite:, 75008 Paris (FR)
(72) Inventeur: GUILLEMET, Alain, F-21121 Fontaine-lès-Dijon (FR)
(74) Mandataire: Clisci, Serge
(86) Numéro de dépôt international: FR9300021
(87) Numéro de publication internationale: WO9314726

(56) Documents cités:
- EP-A- 0 264 299
- EP-A- 0 358 412
- US-A- 4 909 244

## Description

### DOMAINE DE L'INVENTION

La présente invention concerne un procédé de fabrication d'un pansement à bords amincis.

### ART ANTERIEUR

On connaît de EP-A-0 264 299 un pansement du type comprenant un support et un matériau adhésif constitué d'une seule couche adhésive, dans lequel les bords sont biseautés de façon à ce que l'épaisseur extérieure du pansement mesurée à la périphérie n'excède pas 1/4 de l'épaisseur du pansement dans sa partie non amincie. L'avantage essentiel d'un tel pansement est d'éviter le fluage de la masse constitutive du pansement.

Pour la fabrication de ce pansement, il est proposé selon EP-A-0 264 299 de biseauter les bords du pansement dans des conditions drastiques, en appliquant à la périphérie d'une galette constituée par le support revêtu de la couche adhésive une "pression de 10 à 40 tonnes métriques" et une température de 90 à 110°C pendant 1 à 3 secondes.

On connaît de EP-A-0 358 412, un pansement à bords amincis, présentant un profil en escalier de la base au sommet et comprenant trois ensembles distincts. Ces ensembles sont, selon la description (voir colonne 2 ligne 50 à colonne 3 ligne 45), les figures (1 et 2) et les références numériques (reproduites ci-après) de EP-A-0 358 412, les suivants :
(a) au niveau de la base, un premier ensemble constitué par un support non-tissé (référencé 10) revêtu sur sa face de dessus d'une couche adhésive (référencée 11) ;
(b) au-dessus du premier ensemble mais de dimensions (longueur et largeur) plus petites que celui-ci, un second ensemble constitué par un filin (référencé 12) qui est lié par sa face inférieure au premier ensemble par l'intermédiaire de la couche adhésive (référencée 11) dudit premier ensemble, d'une part, et qui est revêtu d'une couche adhésive (référencée 16) sur sa face de dessus, d'autre part ; et
(c) au sommet et au-dessus du second ensemble mais de dimensions (longueur et largeur) plus petites que celui-ci, un troisième ensemble constitué d'une couche de matériau superabsorbant (référencée 18) enrobée à l'intérieur d'un non-tissé (référencé 20), ledit troisième ensemble étant lié au second ensemble par l'intermédiaire de la couche adhésive (référencée 16) dudit second ensemble.

Le pansement selon EP-A-0 358 412 est, au moment de l'utilisation, lié à la peau par l'intermédiaire des zones exposées des couches adhésives des premier et second ensembles.

Il se trouve que les solutions techniques antérieurement connues ne sont pas pratiques. En effet, la solution proposée par EP-A-0 264 299 pour éviter le fluage implique un traitement drastique en ce qui concerne la compression requise pour le biseautage; et celle proposée par EP-A-0 358 412, qui ne concerne pas la limitation ni la suppression du fluage, présente l'inconvénient d'augmenter l'épaisseur de pansement en son centre, d'une part, et de ne pas être adhésif à son sommet (i.e. sur la face supérieure du troisième ensemble), d'autre part.

### BUT DE L'INVENTION

Selon l'invention, on propose à présent une nouvelle solution technique pour éviter le fluage des constituants du pansement et pour remédier aux inconvénients de l'art antérieur.

Cette nouvelle solution met en oeuvre au moins deux couches adhésives superposées selon une construction décroissante de la base vers le sommet dite en escalier, la couche supérieure de cette construction, c'est-à-dire celle disposée au sommet, étant une couche adhésive.

### OBJET DE L'INVENTION

Selon l'invention, on préconise donc un procédé de préparation d'un pansement à bords amincis comprenant, liés entre eux, un support et un système adhésif pelliculaire d'épaisseur diminuant dans la direction de la périphérie, ledit procédé,
qui comprend la formation d'un système adhésif (10) constitué d'au moins deux couches adhésives (11, 12, 13, 14) qui sont
(i) superposées suivant un ensemble de surfaces décroissantes de la base liée au support (2) vers le sommet, et
(ii) agencées de telle façon que deux couches adhésives adjacentes (11/12, 12/13, 13/14) sont liées entre elles et ont des surfaces différentes, les bords de l'une des deux dites couches adhésives adjacentes étant en retrait par rapport aux bords de l'autre couche adhésive,
la couche adhésive (11) formant la base dudit système adhésif (10), qui est liée au support (2), présentant une surface qui est (a) identique à celle dudit support (2) et (b) supérieure à la surface de chaque autre couche (12, 13, 14) dudit système adhésif (10),
étant caractérisé en ce qu'il comprend pour la préparation d'un pansement à bords amincis comportant les au moins deux couches adhésives (11) et (12), les trois étapes successives consistant à :
(1°) déposer sur un support (2) une première couche adhésive (11), et recouvrir ladite première couche adhésive (11) d'un support temporaire antiadhérent ;
(2°) déposer une seconde couche adhésive (12) sur une couche protectrice antiadhérente (4) finale, et
   si nécessaire recouvrir d'un film souple (3) la face de ladite seconde couche adhésive (12) non revêtue par ladite couche protectrice antiadhérente (4) ; et
(3°) former le pansement par découpe du composite obtenu à l'étape (2°) aux dimensions requises, découper ladite couche protectrice antiadhérente (4), appliquer ledit composite ainsi obtenu sur le composite obtenu à l'étape (1°) après avoir éliminé ledit support temporaire antiadhérent recouvrant ladite première couche adhésive (11), puis découper aux dimensions requises la couche protectrice antiadhérente (4), la première couche adhésive (11) et le support (2) de l'ensemble résultant comprenant ladite couche protectrice antiadhérente (4), ladite seconde couche adhésive (12), ladite première couche adhésive (11) et ledit support (2).

Le pansement à bords amincis obtenu selon ce procédé comporte un système adhésif qui est constitué d'au moins deux couches adhésives qui sont
(i) superposées suivant un ensemble de surfaces décroissantes de la base liée au support vers le sommet, et
(ii) agencées de telle façon que deux couches adhésives adjacentes sont liées entre elles et ont des surfaces différentes, les bords de l'une des deux dites couches adhésives adjacentes étant en retrait par rapport aux bords correspondants de l'autre couche adhésive,
la couche adhésive formant la base dudit système adhésif, qui est liée au support, présentant une surface qui est (a) identique à celle dudit support et (b) supérieure à la surface de chaque autre couche dudit système adhésif.

### BREVE DESCRIPTION DES DESSINS

Dans les dessins annexés,
- la figure 1 représente schématiquement en coupe un pansement à bords amincis selon l'invention comportant quatre couches adhésives ;
- la figure 2 représente schématiquement en coupe un pansement à bords amincis selon l'invention comportant trois couches adhésives, dont les zones exposées sont revêtues d'une couche protectrice pelable ; et
- la figure 3 représente schématiquement en coupe un pansement à bords amincis selon l'invention comportant deux couches adhésives de nature différente séparées l'une de l'autre par un film.

### DESCRIPTION DETAILLEE DE L'INVENTION

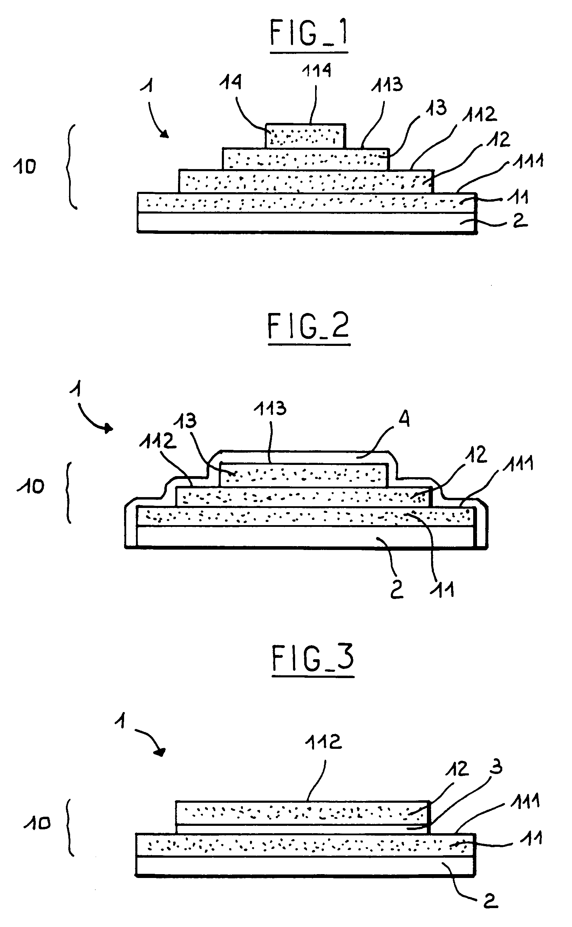

Par référence aux figures 1, 2 et 3, le pansement 1 selon l'invention est du type comprenant un support 2 et un système adhésif 10. Le système adhésif 10 est constitué d'au moins deux couches adhésives (soit en particulier : deux couches adhésives 11, 12 pour le pansement de la figure 3 ; trois couches adhésives 11, 12, 13 pour le pansement de la figure 2 ; et quatre couches adhésives 11, 12, 13, 14 pour le pansement de la figure 1). De façon pratique, le nombre total de couches adhésives utilisées sera de deux, trois ou quatre. En effet, les avantages que pourrait fournir une "construction" comportant plus de quatre couches adhésives ne justifieraient pas l'augmentation des coûts de production en résultant. De préférence, le pansement selon l'invention comprendra deux ou trois couches adhésives.

L'épaisseur du système adhésif 10 diminue en direction de la périphérie, quand on la mesure dans un plan vertical passant par l'axe vertical du pansement. Selon la figure 1, cette épaisseur correspond à la somme des épaisseurs des quatre couches 11-14 dans la portion la plus proche de l'axe du pansement, puis à la somme des épaisseurs des couches inférieures 11-13 et 11-12 quand on s'écarte de l'axe, et enfin à l'épaisseur de la première couche 11 dans la portion la plus éloignée dudit axe.

Par commodité, la couche inférieure 11, qui est également dénommée première couche, est ici celle qui revêt le support 2 [après mise en place du pansement sur la peau, elle sera celle qui est la plus éloignée (au centre du pansement) de la peau], et la couche supérieure 12, 13 ou 14, qui est également dénommée dernière couche, est ici celle qui se trouve au sommet de la construction au profil en escalier du système adhésif 10 [après mise en place du pansement sur la peau, elle sera celle qui est la plus éloignée du support 2 et la plus proche de la peau].

Les couches adhésives sont superposées suivant un ensemble de surfaces décroissantes de la base vers le sommet et agencées de telle façon que deux couches adhésives adjacentes 11/12, 12/13 ou 13/14 soient liées entre elles, les bords de la couche adhésive supérieure 12, 13 ou respectivement 14 étant en retrait par rapport à ceux de la couche inférieure adjacente 11, 12 ou respectivement 13. En d'autres termes, quand on considère deux couches adhésives adjacentes, la surface de la couche supérieure est plus petite que celle de la couche inférieure adjacente. La construction qui résulte de la superposition desdites couches adhésives, fournit au système adhésif 10 et par suite au pansement 1, un profil en escalier décroissant de la base (qui présente la surface la plus grande) vers le sommet (qui présente la surface la plus petite).

La première couche adhésive 11 revêt le support 2 et a une surface identique à celle dudit support. L'égalité des surfaces de la première couche 11 et du support 2 est obtenue par découpe du composite constitué par ledit support 2 revêtu de ladite première couche adhésive 11.

Le support 2 peut être constitué de toute manière connue de l'homme de l'art dans le domaine des pansements, notamment (i) un film de polyuréthanne de grammage compris entre 15 g/m² et 80 g/m², (ii) un non-tissé synthétique de grammage compris entre 5 g/m² et 50 g/m² qui est constitué par exemple de fibres de polyamides, polyesters, polyéthylènes ou polypropylènes, ou encore (iii) un complexe (ou couche composite) formé d'un non-tissé revêtu d'un film de polyuréthanne.

Les couches adhésives 11, 12, 13 et 14 sont chacune constituées d'une masse adhésive sensible à la pression (i.e. autocollante), connue dans le domaine des pansements et de préférence hypoallergénique. Conviennent notamment les masses adhésives acryliques telles que les polymères et copolymères obtenus à partir des monomères acrylate d'alkyle en C₁-C₄ et méthacrylate d'alkyle en C₁-C₄, les masses adhésives telles que les copolymères obtenus à partir des monomères éthylène, propylène et acétate de vinyle, et les mélanges de telles masses. Conviennent également les masses adhésives constituées par des mélanges de copolymères triblocs [du type comprenant un motif unitaire à trois séquences tel que polystyrène-polyoléfine-polystyrène où la séquence centrale (i.e. le fragment polyoléfine) est avantageusement totalement saturé], de plastifiants et, si nécessaire, d'au moins un agent tackyfiant (notamment un matériau acrylate car lesdits copolymères triblocs sont peu ou pas adhésifs), de tels mélanges sont notamment décrits dans la demande FR-A-2 678 513 (publiée le 8 janvier 1993) et les demandes étrangères correspondantes, notamment US No 903 520 (déposée le 24 juin 1992) et européenne EP-A-0 521 761 (publiée le 7 janvier 1993), d'une part, et/ou des mélanges de polymères synthétiques comme par exemple les polyisobutylènes avec des hydrocolloides comme par exemple l'alcool polyvinylique, la pectine, la gélatine ou la carboxyméthylcellulose sodique, d'autre part.

Les couches adhésives 11 et 12, d'une part, et les couches supplémentaires 13 et 14 quand elles sont présentes, d'autre part, peuvent être soit de même nature (c'est-à-dire qu'elles contiennent les mêmes ingrédients essentiels), soit de nature différente (c'est-à-dire qu'elles ne comportent pas les mêmes ingrédients essentiels).

Quand deux couches adhésives adjacentes sont de nature différente, il est recommandé de les séparer par un film 3 intercalaire ayant une surface identique à celle de la couche supérieure qui présente des bords en retrait par rapport aux bords de la surface plus grande de la couche inférieure, comme représenté à la figure 3. Un tel film 3 peut être en polyéthylène, polypropylène ou polyuréthanne à l'instar du support 2.

De façon pratique, si elles sont présentes, les couches adhésives supplémentaires 13 et le cas échéant 14 seront de même nature que la seconde couche adhésive 12, et mieux identiques à ladite seconde couche 12 du point de vue de la composition et de l'épaisseur.

La première couche adhésive 11 et la seconde couche adhésive 12 seront soit de même nature, soit de nature différente, avec dans ce dernier cas, un film 3 de séparation disposé au niveau de leur interface.

De façon avantageuse, chaque couche adhésive 11, 12, 13 ou 14 aura une épaisseur comprise entre 100 et 800 micromètres. De façon préférée, la première couche adhésive 11 aura une épaisseur de 100 à 400 micromètres, la seconde couche adhésive 12 aura une épaisseur de 100 à 800 micromètres quand aucune autre couche adhésive ne lui est superposée et une épaisseur de 100 à 400 micromètres quand une troisième couche 13 lui est superposée, les couches supplémentaires 13 et le cas échéant 14 auront elles une épaisseur de 100 à 400 micromètres quand elles sont présentes.

De façon également préférée, le film 3 aura une épaisseur de 15 à 20 micromètres quand il est réalisé en polyéthylène ou polypropylène, ou une épaisseur de 15 à 25 micromètres quand il est réalisé en polyuréthanne, d'une part, et le support 2 aura une épaisseur de 10 à 50 micromètres et mieux de 15 à 30 micromètres quand il se présente sous la forme d'un film de polyéthylène, polypropylène ou mieux polyuréthanne, d'autre part.

Le profil en escalier permet la fixation à la peau du pansement 1 à bords amincis selon l'invention par l'intermédiaire des zones exposées 111, 112, 113 et 114 des couches adhésives 11, 12, 13 et 14 selon la figure 1 (ou des zones 111, 112 et 113 selon la figure 2 ou encore des zones 111 et 112 selon la figure 3).

De façon également avantageuse, lesdites zones exposées seront revêtues d'une couche protectrice antiadhérente pour leur stockage avant utilisation du pansement à bords amincis selon l'invention. En d'autres termes, le procédé de préparation du pansement 1 comprend le recouvrement au moyen d'une couche protectrice antiadhérente 4 des zones exposées, cette couche protectrice étant temporaire et enlevable ou pelable au moment de la mise en place dudit pansement sur la peau.

Selon un premier mode de réalisation d'un pansement à bords amincis, on préconise de déposer par enduction ou par transfert sur un support 2 une première couche adhésive mince 11 constituée d'une masse acrylique sensible à la pression et hypoallergénique (copolymère d'acrylate d'alkyle en C₁-C₄ ou de méthacrylate d'alkyle en C₁-C₄) puis de superposer par transfert une seconde couche adhésive 12 constituée d'un mélange de copolymère triblocs (selon la demande FR-A-2 678 513 précitée) et de plastifiant et, si nécessaire, d'au moins un agent tackyfiant ou d'un mélange de polymères synthétiques et d'hydrocolloides, de surface inférieure à la surface de la première couche, en ce sens qu'elle est en retrait des bords extérieurs de ladite première couche 11 d'une distance notamment comprise entre environ 0,2 et 1,5 cm.

Selon une première variante de ce premier mode de mise en oeuvre, un film 3 souple de surface identique à la surface de la seconde couche adhésive 12 est intercalé entre la première couche adhésive 11 et ladite seconde couche 12, de façon à laisser exposée la zone 111 de la face supérieure de la masse adhésive de la première couche 11.

Selon une seconde variante dudit premier mode de mise en oeuvre, ladite seconde couche adhésive 12 qui a alors une épaisseur comprise entre 100 et 400 micromètres est revêtue d'une troisième couche adhésive 13. Cette couche adhésive 13 est de même nature et de même épaisseur que ladite seconde couche 12 mais a une surface inférieure à la surface de ladite seconde couche 12, les bords de la troisième couche adhésive 13 étant en retrait des bords extérieurs de la seconde couche adhésive 12 d'une distance notamment comprise entre 0,2 et 1,5 cm.

Selon un second mode de réalisation d'un pansement à bords amincis, on préconise de déposer par enduction ou par transfert sur un support 2 une première couche adhésive mince 11 ayant une épaisseur de 100 à 400 micromètres puis de superposer par transfert une seconde couche adhésive 12 de nature identique à celle de la première couche 11, d'épaisseur comprise entre 100 et 800 micromètres et de surface inférieure à celle de la première couche, les deux couches adhésives 11 et 12 étant notamment constituées d'un mélange de copolymères triblocs, de plastifiants et si nécessaire d'au moins un agent tackyfiant, ou d'un mélange de polymères synthétiques, notamment du type polyisobutylène, et de composés hydrocolloides, et les bords de la seconde couche adhésive 12 étant en retrait par rapport aux bords extérieurs de ladite première couche 11 d'une distance notamment comprise entre environ 0,2 et 1,5 cm.

Le pansement 1 selon l'invention sera avantageusement réalisé selon une forme géométrique simple ayant au moins un axe ou un plan de symétrie. Il sera en particulier :
- inscrit dans une pyramide tronquée, quand sa base (c'est-à-dire la couche adhésive 11 et le support 2) sera polygonale (carrée ou rectangulaire),
- inscrit dans un tronc de cône, quand sa base sera curviligne (notamment circulaire, ovale ou elliptique), ou
- inscrit dans une surface mixte du type pyramide tronquée/tronc de cône, quand sa base comprendra au moins une portion rectiligne et au moins une portion curviligne.

### MEILLEUR MODE

Le meilleur mode de mise en oeuvre du procédé selon l'invention, pour la préparation d'un pansement à bords amincis comportant deux couches adhésives 11 et 12, comprend, d'un point de vue industriel, trois étapes successives consistant à :
(1°) déposer sur un support 2 une première couche adhésive 11, selon une technique d'enduction en phase solvant ou une technique dite à "voie fondue" suivant la nature de ladite première couche adhésive 11, et recouvrir ladite première couche adhésive 11 d'un support temporaire antiadhérent, notamment un papier siliconé ;
(2°) déposer, notamment selon ladite technique dite à "voie fondue", une seconde couche adhésive 12 sur une couche protectrice antiadhérente 4 finale, et
   si nécessaire recouvrir d'un film souple 3 la face de ladite seconde couche adhésive 12 non revêtue par ladite couche protectrice antiadhérente 4 ; et
(3°) former le pansement par découpe du composite obtenu à l'étape (2°) aux dimensions requises, découper ladite couche protectrice antiadhérente 4, appliquer ledit composite ainsi obtenu sur le composite obtenu à l'étape (1°) après avoir éliminé ledit support temporaire antiadhérent recouvrant ladite première couche adhésive 11, puis découper aux dimensions requises la couche protectrice antiadhérente 4, la première couche adhésive 11 et le support 2 de l'ensemble résultant comprenant ladite couche protectrice antiadhérente 4, ladite seconde couche adhésive 12, ladite première couche adhésive 11 et ledit support 2.

Pour préparer un pansement à bords amincis comportant plus de deux couches adhésives, on procède comme indiqué ci-dessus.

D'autres avantages et caractéristiques de l'invention seront mieux compris à la lecture qui va suivre d'exemples de réalisation nullement limitatifs mais donnés à titre d'illustration.

### EXEMPLE 1

On prépare, selon le meilleur mode décrit ci-dessus, un pansement à bords amincis comprenant les couches adhésives 11 et 12. Ce pansement comporte :
- en tant que support 2, un film de polyuréthanne ayant une épaisseur de 30 micromètres ;
- en tant que première couche adhésive 11, une masse acrylique, à base de copolymère acrylate d'éthyle/méthacrylate de méthyle/méthacrylate de 2-hydroxyéthyle, ayant une épaisseur de 250 micromètres ;
- en tant que film 3 de séparation, un film de polyéthylène ayant une épaisseur de 15 micromètres ;
- en tant que seconde couche adhésive 12, une masse adhésive constituée par un mélange (i) de copolymère tribloc polystyrène/polyéthylène-butylène/polystyrène, (ii) de pétrolatum, (iii) de résine de synthèse WINGTACK 95 commercialisée par la société GOODYEAR et (iv) de carboxyméthylcellulose sodique (CMC) [où les moyens (i) et (ii), qui ne sont pas adhésifs, sont ceux décrits à l'exemple 1 du document FR-A-2 678 513 précité, le moyen (iii) fournit le caractère adhésif à ladite masse en raison de son pouvoir tackyfiant et le moyen (iv) fournit un caractère absorbant à ladite masse] et ayant une épaisseur de 450 micromètres ; et
- en tant qu'une couche protectrice antiadhérente 4, une pellicule siliconée.

### EXEMPLE 2

On prépare, selon le meilleur mode décrit ci-dessus, un pansement à bords amincis comprenant trois couches adhésives, à savoir les couches 11, 12 et 13. Ce pansement comporte :
- en tant que support 2, un film de polyuréthanne ayant une épaisseur de 50 micromètres ;
- en tant que première couche adhésive 11, une masse acrylique, à base de copolymère acrylate d'éthyle/méthacrylate de méthyle ayant une épaisseur de 200 micromètres ;
- en tant que film 3 de séparation, un film de polyuréthanne ayant une épaisseur de 20 micromètres ;
- en tant que seconde couche adhésive 12, la masse adhésive constituée par le mélange copolymère tribloc/pétrolatum/résine de synthèse/CMC décrit à l'exemple 1 ci-dessus et ayant une épaisseur de 250 micromètres ;
- en tant que troisième couche adhésive 13, la masse adhésive identique par sa composition et son épaisseur à celle de la seconde couche adhésive 12 qu'elle revêt ;
- en tant que une couche protectrice antiadhérente 4, une pellicule siliconée.

### EXEMPLE 3

On prépare comme indiqué ci-dessus pour l'exemple 2, un pansement à bords amincis comprenant trois couches adhésives 11, 12 et 13. Ce pansement est identique à celui de l'exemple 2 avec la différence que (i) la première couche adhésive 11 est une masse adhésive constituée par le mélange copolymère tribloc/pétrolatum/résine de synthèse/CMC décrit à l'exemple 1 ci-dessus et ayant une épaisseur de 400 micromètres ; (ii) les couches adhésives 12 et 13, identiques aux couches 12 et 13 de l'exemple 2, ont chacune une épaisseur de 300 micromètres et (iii) le film souple 3 a été omis entre les couches adhésives 11 et 12.

### EXEMPLE 4

On prépare comme indiqué ci-dessus pour l'exemple 2, un pansement à bords amincis ne comprenant que deux couches adhésives 11 et 12. Les deux couches adhésives qui ont la même composition sont constituées par le mélange copolymère tribloc/pétrolatum/résine de synthèse/CMC, la première couche 11 ayant une épaisseur de 400 micromètres et la seconde couche 12 ayant une épaisseur de 600 micromètres, les deux couches 11 et 12 n'étant pas séparées par un film souple.

## Revendications

1. Procédé de préparation d'un pansement (1) à bords amincis comprenant, liés entre eux, un support (2) et un système adhésif pelliculaire (10) d'épaisseur diminuant dans la direction de la périphérie, ledit procédé, qui comprend la formation d'un système adhésif (10) constitué d'au moins deux couches adhésives (11, 12, 13, 14) qui sont
(i) superposées suivant un ensemble de surfaces décroissantes de la base liée au support (2) vers le sommet, et
(ii) agencées de telle façon que deux couches adhésives adjacentes (11/12, 12/13, 13/14) sont liées entre elles et ont de surfaces différentes, les bords de l'une des deux dites couches adhésives adjacentes étant en retrait par rapport aux bords de l'autre couche adhésive,
la couche adhésive (11) formant la base dudit système adhésif (10), qui est liée au support (2), présentant une surface qui est (a) identique à celle dudit support (2) et (b) supérieure à la surface de chaque autre couche (12, 13, 14) dudit système adhésif (10),
étant caractérisé en ce qu'il comprend, pour la préparation d'un pansement à bords amincis comportant les au moins deux couches adhésives (11) et (12), les trois étapes successives consistant à :
(1°) déposer sur un support (2) une première couche adhésive (11), et recouvrir ladite première couche adhésive (11) d'un support temporaire antiadhérent ;
(2°) déposer une seconde couche adhésive (12) sur une couche protectrice antiadhérente (4) finale, et
si nécessaire recouvrir d'un film souple (3) la face de ladite seconde couche adhésive (12) non revêtue par ladite couche protectrice antiadhérente (4) ; et
(3°) former le pansement par découpe du composite obtenu à l'étape (2°) aux dimensions requises, découper ladite couche protectrice antiadhérente (4), appliquer ledit composite ainsi obtenu sur le composite obtenu à l'étape (1°) après avoir éliminé ledit support temporaire antiadhérent recouvrant ladite première couche adhésive (11), puis découper aux dimensions requises la couche protectrice antiadhérente (4), la première couche adhésive (11) et le support (2) de l'ensemble résultant comprenant ladite couche protectrice antiadhérente (4), ladite seconde couche adhésive (12), ladite première couche adhésive (11) et ledit support (2).

2. Procédé suivant la revendication 1, dans lequel la formation dudit système adhésif (10) met en oeuvre une première couche adhésive (11) formant la base dudit système adhésif (10) et une seconde couche adhésive (12) qui sont de même nature.

3. Procédé suivant la revendication 1, dans lequel la formation dudit système adhésif (10) met en oeuvre une première couche adhésive (11) formant la base dudit système adhésif (10) et une seconde couche adhésive (12) qui sont de nature différente.

4. Procédé suivant la revendication 1, dans lequel la ou les couches adhésives supplémentaires (13, 14), qui forment avec les première et seconde couches adhésives (11, 12) ledit système adhésif (10), sont de même nature que ladite seconde couche adhésive (12).

5. Procédé suivant la revendication 3, dans lequel un film souple (3) est prévu pour séparer lesdites première et seconde couches adhésives (11, 12) quand celles-ci sont de nature différente, ledit film (3) ne recouvrant pas la zone (111) de la première couche adhésive (11) exposée par la superposition de la seconde couche adhésive (12).

6. Procédé suivant la revendication 1, dans lequel toutes les couches adhésives (11, 12, 13, 14) dudit système adhésif (10) sont de même nature.

7. Procédé suivant la revendication 1, caractérisé en ce qu'il comprend le recouvrement, au moyen d'une couche protectrice antiadhérente (4), des zones exposées (111, 112, 113, 114) des couches adhésives (11, 12, 13, 14) dudit système adhésif (10).

## Claims

1. A method for the preparation of a dressing (1) with thinned edges comprising, joined to one another, a support (2) and a film-type adhesive system (10) of reducing thickness in the direction of the periphery, said method, which comprises the formation of an adhesive system (10) consisting of at least two adhesive layers (11, 12, 13, 14) which are
(i) superimposed to form a set of reducing surface areas from the base, linked to the support (2), to the top, and
(ii) arranged in such a way that two adjacent adhesive layers (11/12), 12/13, 13/14) are joined together and have different surface areas, the edges of one of the two said adjacent adhesive layers being offset relative to the edges of the other adhesive layer,
the adhesive layer (11) forming the base of said adhesive system (10), which is linked to the support (2), having a surface area which is (a) identical to that of said support (2) and (b) greater than the surface area of every other layer (12, 13, 14) of said adhesive system (10),
being characterized in that it comprises for the preparation of a dressing with thinned edges containing at least the two adhesive layers (11) and (12), three successive steps consisting in :
(1°) placing a first adhesive layer (11) on a support (2) and covering said first adhesive layer (11) with a non-stick temporary support ;
(2°) placing a second adhesive layer (12) on a final non-stick protective layer (4) and, if necessary, covering with a flexible film (3) the face of said second adhesive layer (12) which is not covered with said non-stick protective layer (4) ; and
(3°) forming the dressing by cutting the composite obtained in step (2°) to the requisite dimensions, cutting said non-stick protective layer (4), applying said composite thus obtained to the composite obtained in step (1°) after removal of said non-stick temporary support covering said first adhesive layer (11), and then cutting to the requisite dimensions the non-stick protective layer (4), the first adhesive layer (11) and the support (2) of the resulting assembly comprising said non-stick protective layer (4), said second adhesive layer (12), said first adhesive layer (11) and said support (2).

2. A method according to claim 1 in which the formation of said adhesive system (10) involves a first adhesive layer (11) forming the base of said adhesive system (10), and a second adhesive layer (12), which are of the same type.

3. A method according to claim 1 in which the formation of said adhesive system (10) involves a first adhesive layer (11) forming the base of said adhesive system (10), and a second adhesive layer (12), which are of different types.

4. A method according to claim 2 or 3 in which the additional adhesive layers (13, 14), which form said adhesive system (10) with the first and second adhesive layers (11, 12), are of the same type as said second adhesive layer (12).

5. A method according to claim 3 in which a flexible film (3) is provided in order to separate said first and second adhesive layers (11, 12) when they both are of different types, said film (3) not covering the zone (111) of the first adhesive layer (11) which is exposed by the superimposition of the second adhesive layer (12).

6. A method according to claim 1 in which all the adhesive layers (11, 12, 13, 14) of said adhesive system (10) are of the same type.

7. A method according to claim 1 which comprises covering with a non-stick protective layer (4) the exposed zones (111, 112, 113, 114) of the adhesive layers (11, 12, 13,14) of said adhesive system (10).

## Patentansprüche

1. Verfahren zur Herstellung eines Verbandes (1) mit dünnen Rändern, bestehend aus einer Unterlage (2) und einem hautartigen Klebstoffsystem (10) mit in Richtung des Umfangs abnehmender Dicke, die miteinander verbunden sind, das die Bildung eines Klebstoffsystems (10) umfaßt, bestehend aus wenigstens zwei Klebstoffschichten (11, 12, 13, 14), die
(i) entsprechend einer Anordnung von Flächen, die von der Basis, die mit der Unterlage (2) verbunden ist, zur Spitze abnehmen, übereinander liegen, und
(ii) derart angeordnet sind, daß zwei benachbarte Klebstoffschichten (11/12, 12/13, 13/14) untereinander verbunden sind und unterschiedliche Flächen haben, wobei die Ränder einer der beiden benachbarten Klebstoffschichten bzgl. der Ränder der anderen Klebstoffschicht zurückgesetzt sind,
wobei die Klebstoffschicht (11), die die Basis des Klebstoffsystems (10) bildet, die mit der Unterlage verbunden ist, eine Fläche hat, die (a) gleich der der Unterlage (2) ist, und (b) größer als die Fläche jeder anderen Schicht (12, 13, 14) des Klebstoffsystems ist,
**dadurch gekennzeichnet, daß**
es zur Herstellung eines Verbandes mit dünnen Rändern, der die wenigstens zwei Klebstoffschichten (11) und (12) aufweist, die drei folgenden Schritte umfaßt, die bestehen aus:
(1.) dem Auflegen einer ersten Klebstoffschicht (11) auf eine Unterlage (2) und dem Abdecken der ersten Klebstoffschicht (11) mit einer provisorischen Antihaftunterlage;
(2.) dem Auflegen einer zweiten Klebstoffschicht (12) auf eine Antihaft-Endschutzschicht (4) und nötigenfalls dem Abdecken der Oberseite der zweiten Klebstoffschicht (12), die nicht durch die Antihaftschutzschicht (4) überzogen ist, mit einem flexiblen Film; und
(3.) dem Formen des Verbandes durch Zuschneiden des Schichtkörpers, der beim zweiten Schritt erhalten wird, auf die erforderlichen Abmessungen, dem Zuschneiden der Antihaftschutzschicht (4), dem Auflegen des so erhaltenen Schichtkörpers auf den beim ersten Schrift erhaltenen Schichtkörper, nachdem die provisorische Antihaftunterlage entfernt wurde, die die erste Klebstoffschicht (11) abdeckt, dann dem Zuschneiden auf die erforderlichen Abmessungen der Antihaftschutzschicht (4), der ersten Klebstofkchicht (11) und der Unterlage (2) der sich ergebenden Anordnung, die die Antihaftschutzschicht (4), die zweite Klebstoffschicht (12), die erste Klebstoffschicht (11) und die Unterlage (2) umfaßt.

2. Verfahren nach Anspruch 1, bei dem die Bildung des Klebstoffsystems (10) eine erste Klebstoffschicht (11), das die Basis des Klebstoffsystems (10) bildet, und eine zweite Klebstoffschicht (12) umfaßt, die von derselben Art sind.

3. Verfahren nach Anspruch 1, bei dem die Bildung des Klebstoffsystems (10) eine erste Klebstoffschicht (11) umfaßt, die die Basis des Klebstoffsystems (10) bildet, und eine zweite Klebstoffschicht (12), die unterschiedlicher Art sind.

4. Verfahren nach Anspruch 1, bei dem die zusätzliche(n) Klebstoffschicht(en) (13, 14), die zusammen mit der ersten und zweiten Klebstoffschicht (11, 12) das Klebstoffsystem (10) bilden, von der gleichen Art wie die zweite Klebstoffschicht (12) sind.

5. Verfahren nach Anspruch 3, bei dem ein flexibler Film (3) vorgesehen ist, um die erste und zweite Klebstoffschicht (11, 12) zu trennen, wenn diese gleicher Art sind, wobei der Film (3) den Bereich (111) der ersten Klebstoffschicht (11) bedeckt, der durch Überlagerung der zweiten Klebstoffschicht (12) freiliegt.

6. Verfahren nach Anspruch 1, bei dem alle Klebstoffschichten (11, 12, 13, 14) des Klebstoffsystems (10) von der gleichen Art sind.

7. Verfahren nach Anspruch 1,
**dadurch gekennzeichnet, daß**
es die Abdeckung mittels wenigstens einer Antihaftschutzschicht (4) der freiliegenden Bereiche (111, 112, 113, 114) der Klebstoffschichten (11, 12, 13, 14) des Klebstoffsystems (10) umfaßt.
